# EUROPEAN PATENT APPLICATION

(11) **EP 2 063 253 A2**
(43) Date of publication of application: **27.05.2009**
(21) Application number: 08020456.3
(22) Date of filing: 25.11.2008
(51) Int. Cl.: G01N 21/35

(54) **Method for identifying microorganism or detecting its morphology alteration using surface enhanced ramam scattering (SERS)**

(30) Priority: 26.11.2007 US 945101
(71) Applicant: National Yang-Ming University, Beitou District Taipei City 112 (TW)
(72) Inventor: Lin, Chi-Hung, Taipei City 112 (TW); Liu, Ting-Ting, Taipei City 112 (TW); Huang, Yung-Ching, Taipei City 112 (TW); Wang, Huai-Hsien, Taipei City 112 (TW); Wang, Juen-Kai, Taipei City 112 (TW); Wang, Yuh-Lin, Taipei City 112 (TW)
(74) Representative: Merkle, Gebhard

(57) **Abstract**

The present invention relates to a method for producing a profile identifying a microorganism based on surface enhanced Raman scattering (SERS) and an apparatus thereof. The method comprises: (1) placing the microorganism on a SERS-active substrate; (2) mounting the microorganism with a mounting solution; (3) obtaining a SERS spectrum of the microorganism in step (2); and (4) analyzing the SERS spectrum to produce the profile.

The present invention also relates to a method for detecting morphology alteration of a microorganism due to an antimicrobial agent or an infectious agent based on SERS and an apparatus thereof. The method comprises: (1) placing the microorganism on a SERS-active substrate; (2) mounting the microorganism with a mounting solution; (3) treating the microorganism with a pharmaceutically effective amount of the antimicrobial agent or the infectious agent; (4) obtaining a SERS spectrum of the microorganism in step (3); and (5) identifying effect of the antimicrobial agent or the infectious agent by at least one new peak in the SERS spectrum in step (4) compared to a SERS spectrum of a control sample, wherein the control sample is not treated with the antimicrobial agent or the infectious agent.

## Description

### FIELD OF THE INVENTION

The present invention relates to a method and an apparatus for producing a profile identifying a microorganism based on surface enhanced Raman scattering (SERS). The present invention also relates to a method and an apparatus for detecting morphology alteration of a microorganism due to an antimicrobial agent or an infectious agent based on SERS. The present invention further provides a method for detecting *Mycobacterium tuberculosis* in clinical examination.

### BACKGROUND OF THE INVENTION

Raman spectroscopy is attracting interest for the rapid identification of bacteria and fungi and is now becoming accepted as a potentially powerful whole-organism fingerprinting technique. Jarvis *et al* investigated enhanced Raman scattering (SERS), employing an aggregated silver colloid substrate, for the analysis of a closely related group of bacteria belonging to the genus *Bacillus.* (R. M. Jarvis, A. Brookerb and R. Goodacre, Faraday Discuss., 2006, 132, 281-292.) The results showed that the SERS spectra were highly discriminatory and gave accurate identification at the strain level.

However, the reproducibility and stability of the method utilizing colloid substrate is not as good as that of the method using a substrate with a solid film embedded or covered with nanoparticles.

In the study of Premasiri *et al,* the SERS of a number of species and strains of bacteria obtained on novel gold nanoparticle (~80 mn) covered SiO₂ substrates excited at 785 nm was reported (W. R. Premasiri, D. T. Moir, M. S. Klempner, N. Krieger, G. Jones II, and L. D. Ziegler, J. Phys. Chem. B 2005, 109, 312-320). The results revealed how the SERS vibrational signatures are strongly dependent on the morphology and nature of the SERS active substrates. The potential of SERS for detection and identification of bacterial pathogens with species and strain specificity on these gold particle covered glassy substrates were demonstrated by these results.

Those skilled in the art would realize that according to the method of Premasiri *et al,* it was necessary to dry the bacterial suspension on the solid substrate before obtaining SERS signals. Otherwise, the movement of bacteria would interfere with the result.

In recent years the number of new antimicrobial agents has decreased dramatically, with concomitant increase of pathogen that became multi-drug resistant. It is a significant challenge to discover new antibiotics with the advanced technologies that characterize the mode of action of pharmacological materials rapidly and reliably. Monitoring effects of antibiotics on bacteria using SERS should be a powerful approach for the development and screening of new antibiotics. To achieve that, the bacteria need to be living in an aqueous environment in order to be exposed to the antibiotics.

Tuberculosis (TB) is a common and deadly infectious disease caused by mycobacteria, mainly *Mycobacterium tuberculosis.* Tuberculosis most commonly attacks the lungs but can also affect the central nervous system, the lymphatic system, the circulatory system, the genitourinary system, bones, joints and even the skin. At present, the diagnosis for TB includes a tuberculin skin test, a serological test, microbiological smears and cultures a chest X-ray, , and PCR.

The examination methods used so far do not meet the needs in the clinical practice where speedy detection of tubercle bacillus or speedy confirmation of the treatment is required. Accordingly, a more rapid and highly sensitive detection is desired.

### SUMMARY OF THE INVENTION

The present invention relates to a method for producing a profile identifying a microorganism based on surface enhanced Raman scattering (SERS). The method comprises: (a) placing the microorganism on a SERS-active substrate; (b) mounting the microorganism with a mounting solution; (c) obtaining a SERS spectrum of the microorganism in step (b); and (d) analyzing the SERS spectrum to produce a profile of the microorganism.

The present invention also relates to a method for detecting morphology alteration of a microorganism due to an antimicrobial agent or an infectious agent based on SERS. The method comprises: (a) placing the microorganism on a SERS-active substrate; (b) mounting the microorganism with a mounting solution; (c) treating the microorganism with a pharmaceutically effective amount of the antimicrobial agent or the infectious agent; (d) obtaining a SERS spectrum of the microorganism in step (c); and (e) identifying effect of the antimicrobial agent or the infectious agent by at least one new peak in the SERS spectrum in step (d) compared to a SERS spectrum of a control sample, wherein the control sample is not treated with the antimicrobial agent or the infectious agent.

The present invention also relates to an apparatus for producing a profile of a microorganism based on surface enhanced Raman scattering (SERS), comprising: (a) means of placing the microorganism on a SERS-active substrate; (b) means of mounting the microorganism; (c) means of obtaining a SERS spectrum of the microorganism in step (b); and (d) means of analyzing the SERS spectrum to produce a profile of the microorganism.

The present invention also relates to an apparatus for detecting morphology alteration of a microorganism due to an antimicrobial agent or an infectious agent based on surface enhanced Raman scattering (SERS), comprising: (a) means of placing the microorganism on a SERS-active substrate; (b) means of mounting the microorganism; (c) means of treating the microorganism with a pharmaceutically effective amount of the antimicrobial agent or the infectious agent; (d) means of obtaining a SERS spectrum of the microorganism in step (c); and (e) means of identifying effect of the antimicrobial agent or the infectious agent by at least one new peak in the SERS spectrum in step (d) compared to a SERS spectrum of a control sample, wherein the control sample is not treated with the antimicrobial agent or the infectious agent.

The present invention further provides a method for detecting *Mycobacterium tuberculosis* infection, comprising: (a) placing the sample on a SERS-active substrate; (b) mounting the microorganism with a mounting solution; (c) obtaining a SERS spectrum of the sample in step (b); (d)analyzing the SERS spectrum to produce a profile of the sample; and (e)comparing the SERS spectrum of the sample with the SERS spectrum of gram-positive and gram-negative bacteria.

### BRIEF DESCRIPTION OF THE DRAWINGS

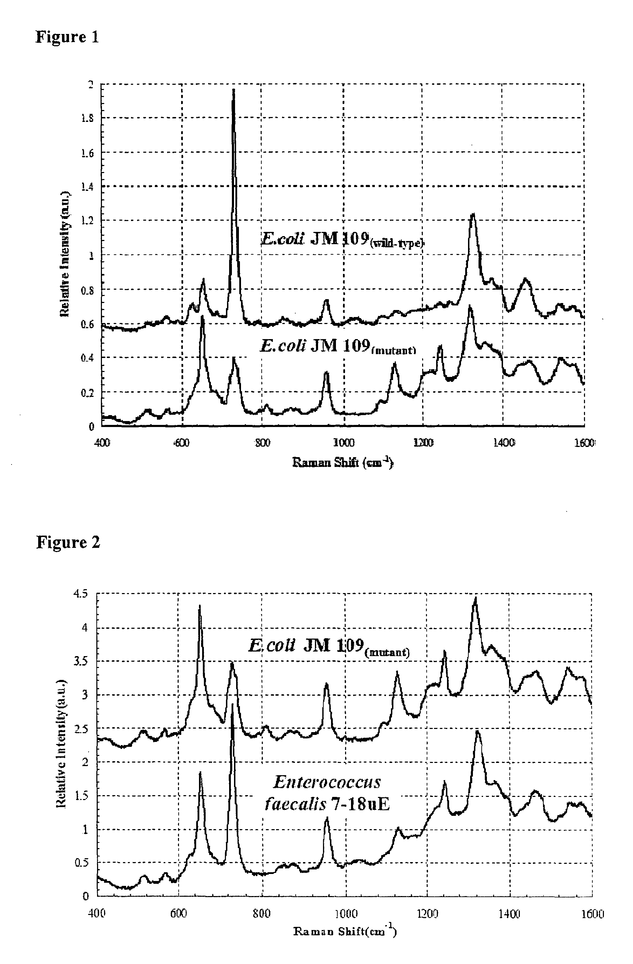

Figure 1 illustrates the comparison of SERS spectra of the strain *Escherichia coli* JM109 and its mutant *Escherichia coli* JM109₍ₘᵤₜₐₙₜ₎.
Figure 2 illustrates the comparison of SERS spectra of the strain *Escherichia coli* JM109₍ₘᵤₜₐₙₜ₎ and the strain *Enterococcus faecalis* 7-18uE.

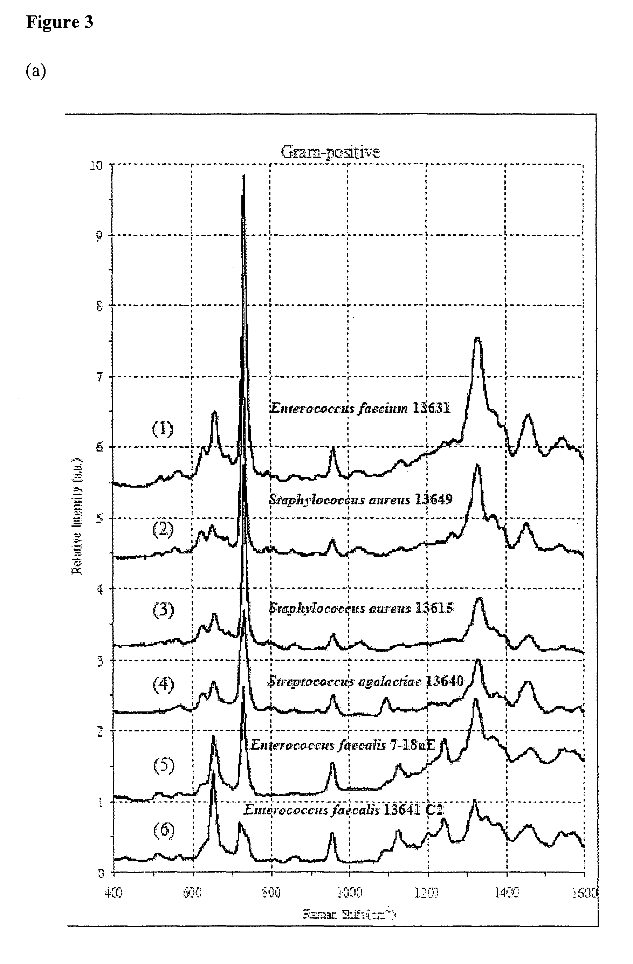

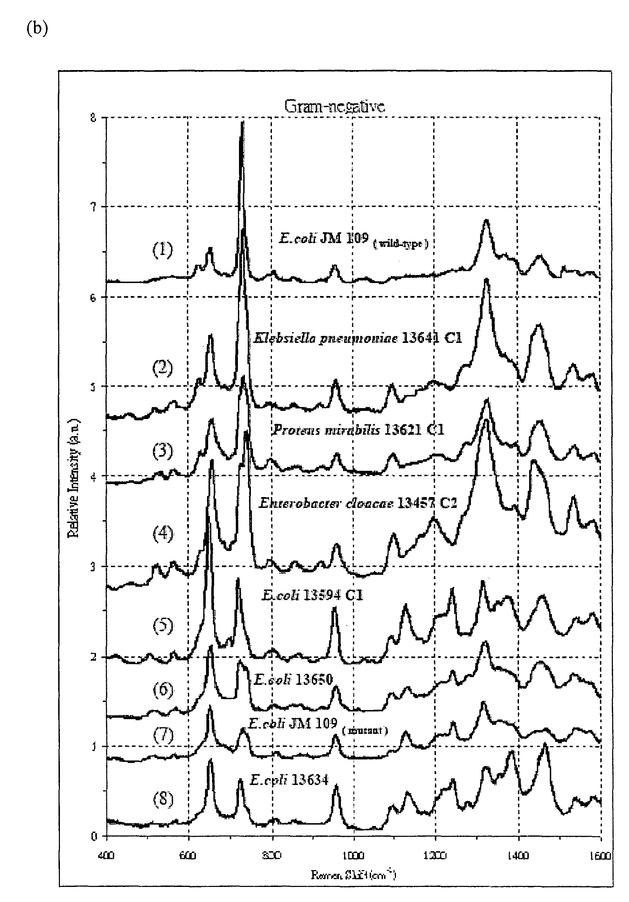

Figure 3 illustrates the SERS spectra of the Gram-positive bacteria and Gram-negative bacteria. (a) shows the spectra of Gram-positive bacteria *(Enterococcus faecium* 13631, *Staphylococcus aureus* 13649, *Staphylococcus aureus* 13615, *Streptococcus agalactiae* 13640, *Enterococcus faecalis* 7-18uE, *Enterococcus faecalis* 13641 C2). (b) shows the spectra of Gram-negative bacteria *(Escherichia coli* JM109_{(wild-type)}, *Klebsiella pneumoniae* 13641 C1, *Proteus mirabilis* 13621 C1, *Enterobacter cloacae* 13457 C2, *E. coli* 13594 C1, *E. coli* 13650, *Escherichia coli* JM109₍ₘᵤₜₐₙₜ₎, *E. coli* 13634).

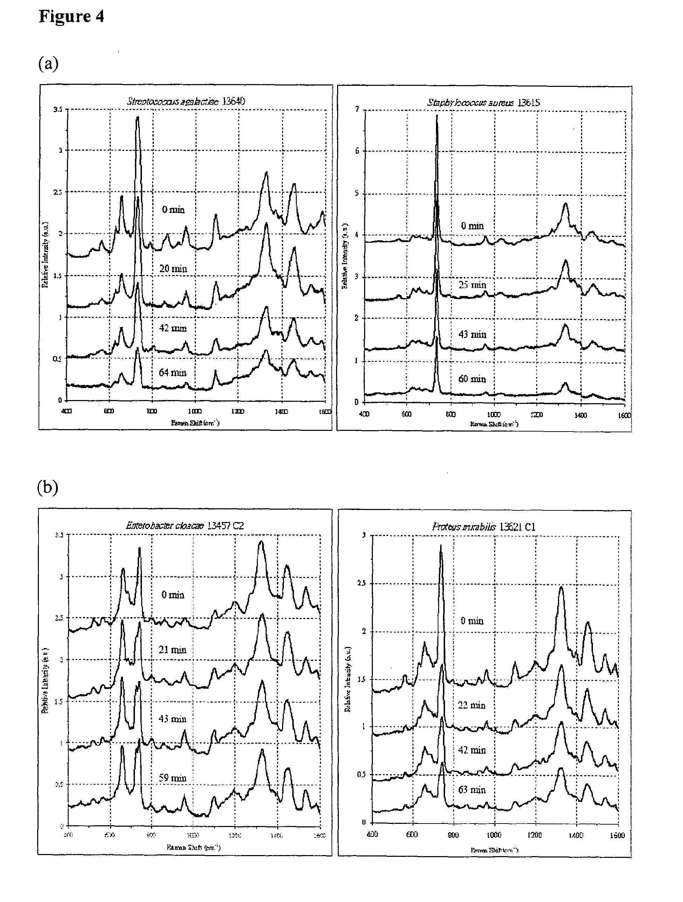

Figure 4 shows serial detection of the SERS spectra of Gram-positive bacteria *(Streptococcus agalactiae* 13640 and *Staphylococcus aureus* 13615) and Gram-negative bacteria *(Enterobacter cloacae* 13457 C2 and *Proteus mirabilis* 13621 C1). (a) shows the spectra of Gram-positive bacteria (*Streptococcus agalactiae* 13640 and *Staphylococcus aureus* 13615). (b) shows the spectra of Gram-negative bacteria (*Enterobacter cloacae* 13457 C2 and *Proteus mirabilis* 13621 C1).

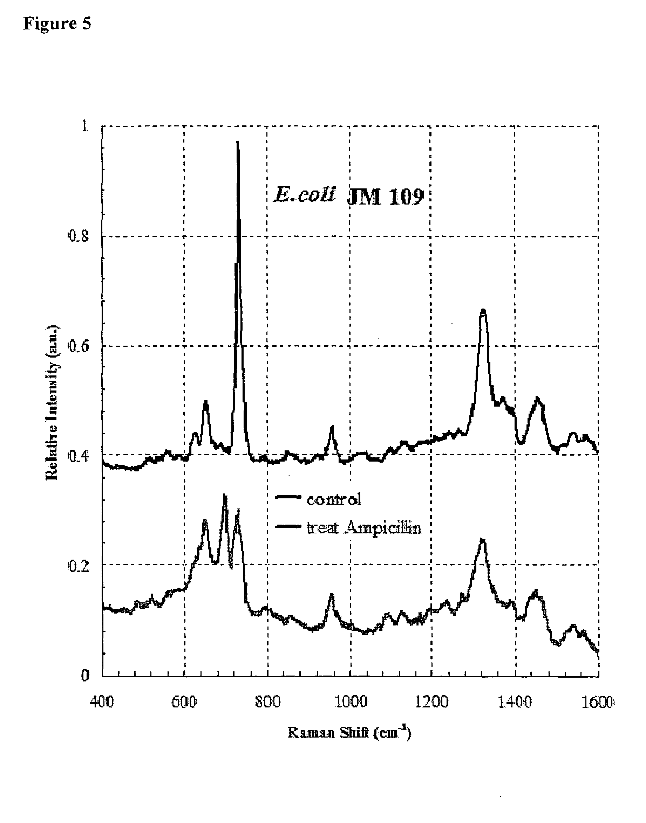

Figure 5 shows the SERS spectra of the antibiotic susceptible strain *Escherichia coli* JM109. The presence of a new peak at 700 cm⁻¹ in the spectrum of JM109 treated with 10µg/ml ampicillin for 35 min compared with that of JM109 with no treatment demonstrates the oscillation of a new molecular bond caused by ampicillin.

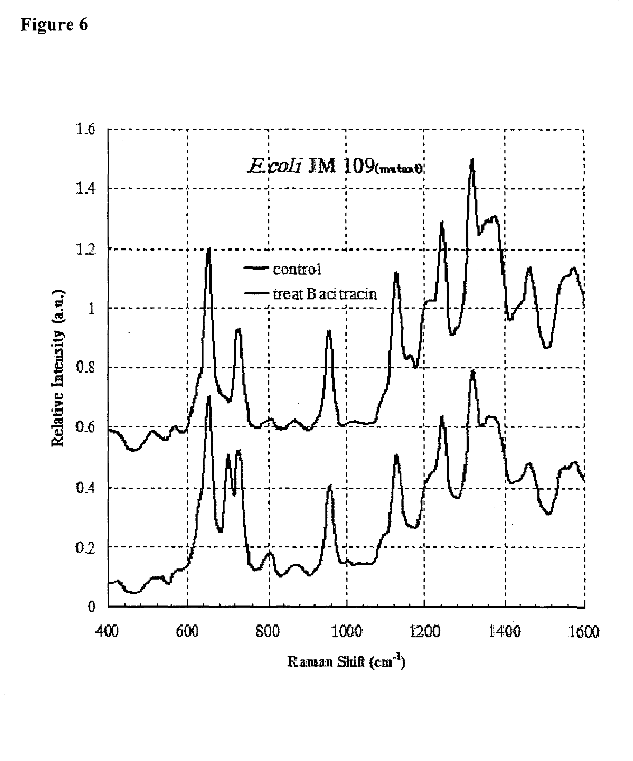

Figure 6 shows the SERS spectra of the antibiotic susceptible strain *Escherichia coli* JM109₍ₘᵤₜₐₙₜ₎. The presence of a new peak at 700 cm⁻¹ in the spectrum of JM109₍ₘᵤₜₐₙₜ₎ treated with 25 µg/ml bacitracin for 48 min compared with that of JM109₍ₘᵤₜₐₙₜ₎ with no treatment demonstrates the oscillation of a new molecular bond caused by bacitracin.

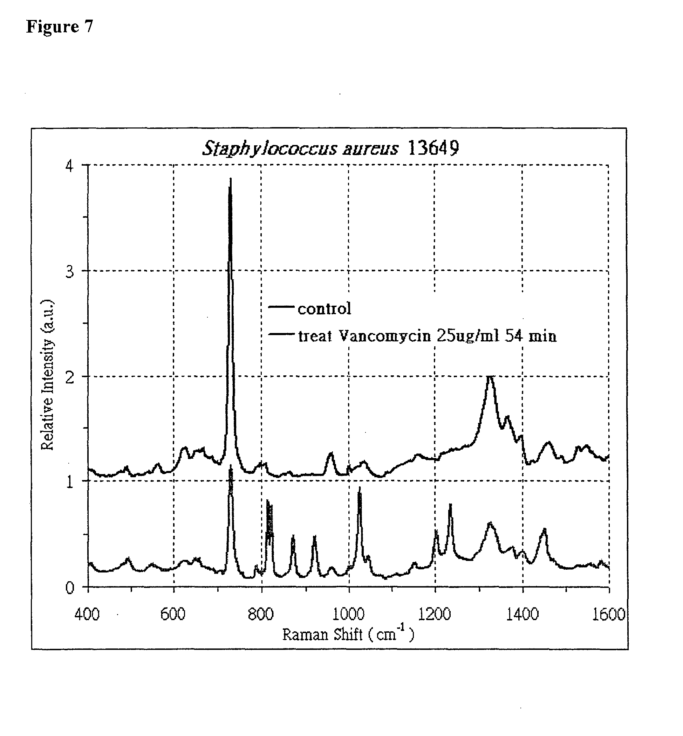

Figure 7 shows the SERS spectra of the antibiotic susceptible strain *Staphylococcus aureus* 13649. The presence of new peaks at 800 cm⁻¹ to 1500 cm⁻¹ after treating with 25µg/ml vancomycin for 54 min demonstrates the oscillation of a new molecular bond caused by vancomycin.

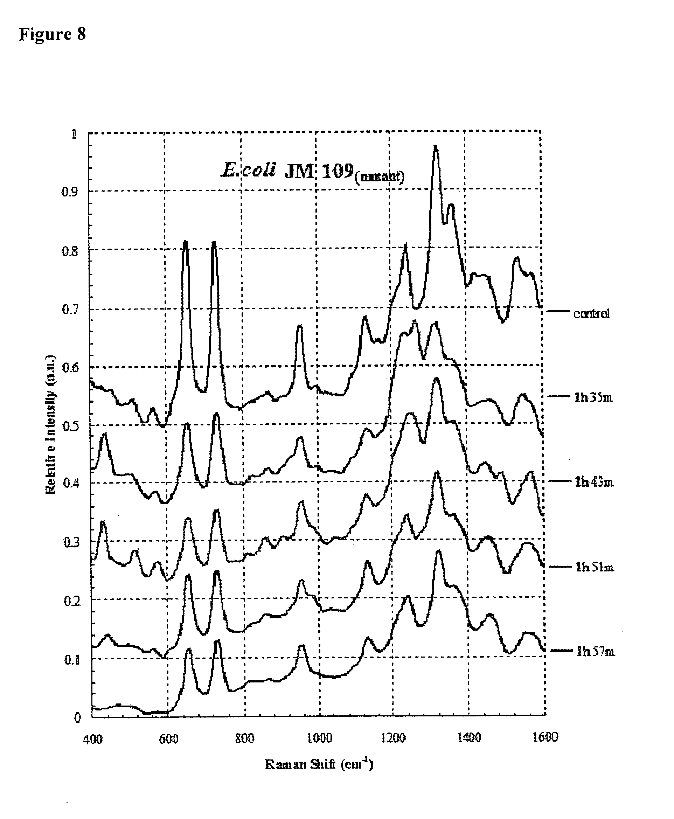

Figure 8 shows the SERS spectra of the antibiotic susceptible strain *Escherichia coli* JM109₍ₘᵤₜₐₙₜ₎. The presence of new peaks at 440 cm⁻¹ and 1242 cm⁻¹ in the spectrum of JM109₍ₘᵤₜₐₙₜ₎ treated with 50µg/ml tetracycline compared with that of JM109₍ₘᵤₜₐₙₜ₎ with no treatment demonstrates the oscillations of new molecular bonds caused by tetracycline at different time points.

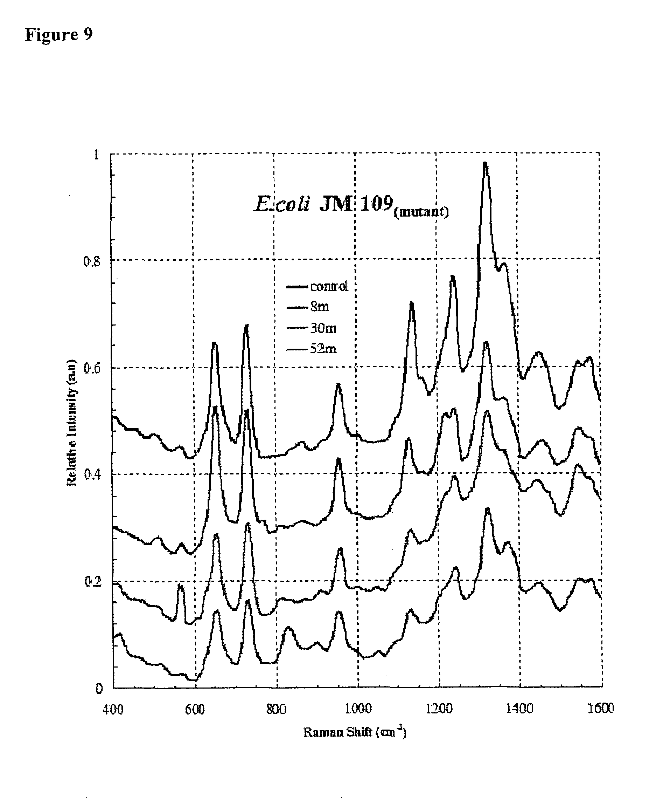

Figure 9 shows the SERS spectra of the antibiotic susceptible strain *Escherichia coli* JM109₍ₘᵤₜₐₙₜ₎. The presence of new peaks at 418 cm⁻¹, 565 cm⁻¹, 833 cm⁻¹, 905 cm⁻¹ and 1225 cm⁻¹ in the spectrum of JM109₍ₘᵤₜₐₙₜ₎ treated with 50µg/ml tetracycline compared with that of JM109₍ₘᵤₜₐₙₜ₎ with no treatment demonstrates the oscillations of several new molecular bonds caused by tetracycline at different time points.

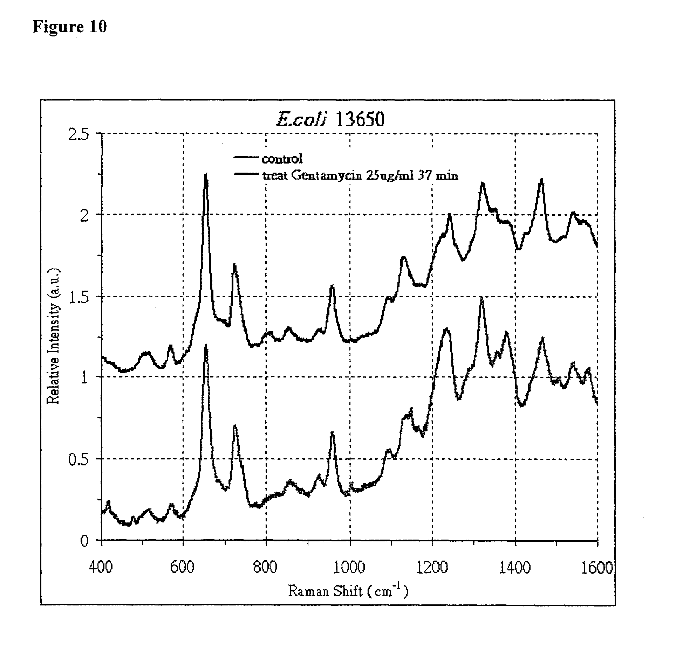

Figure 10 shows the SERS spectra of the antibiotic susceptible strain *E. coli* 13650. The presence of new peaks at 418 cm⁻¹ and at 800 cm⁻¹ to 1500 cm⁻¹ after treating with 25µg/ml gentamycin for 37 min demonstrates the oscillation of a new molecular bond caused by gentamycin.

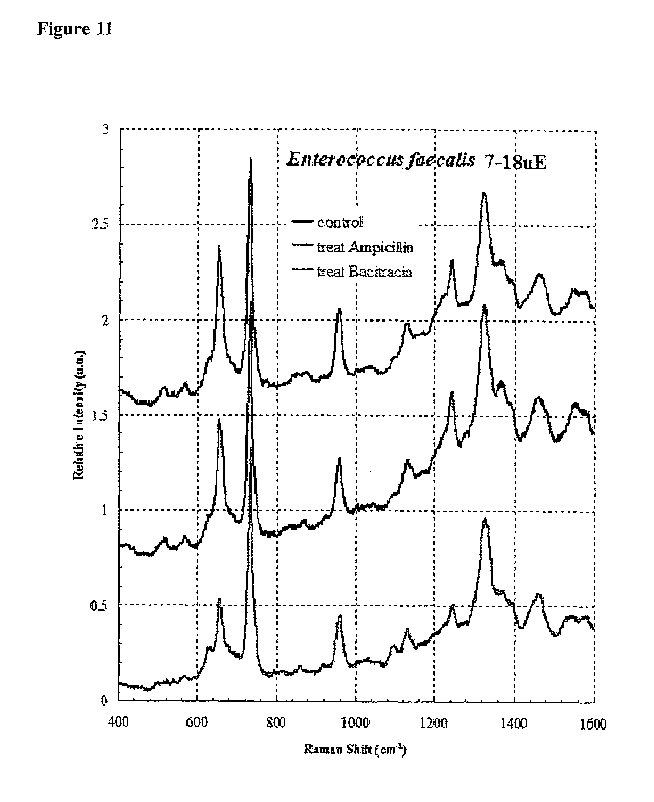

Figure 11 shows the SERS spectra of the antibiotic resistant strain *Enterococcus faecalis* 7-18uE. No spectral changes were observed in the spectra of 7-18uE treated with ampicillin and bacitracin, demonstrating the drug resistance of 7-18uE.

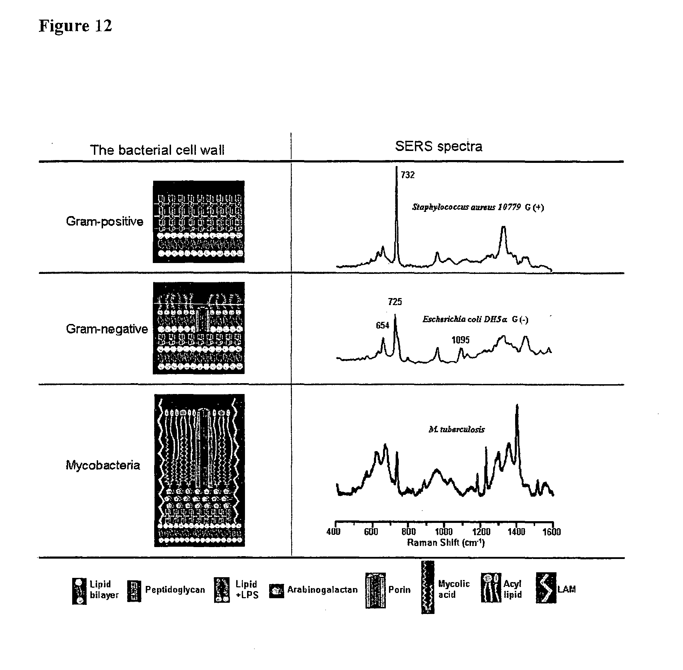

Figure 12 show the SERS spectra of the gram-positive, gram-negative and mycobacteria.

### DETAILED DESCRIPTION OF THE INVENTION

The goals of the invention are: (1) distinguishing microorganism species and strains; (2) identification of the effects of antibiotics or infectious agents on bacterial cells; and (3) detecting TB infection.

The present invention relates to a method for producing a profile identifying a microorganism based on surface enhanced Raman scattering (SERS). The method comprises:
(a) placing the microorganism on a SERS-active substrate;
(b) mounting the microorganism with a mounting solution;
(c) obtaining a SERS spectrum of the microorganism in step (b); and
(d) analyzing the SERS spectrum to produce a profile of the microorganism.

In a preferred embodiment, the SERS-active substrate is a solid film embedded or covered with nanoparticles. Preferably, the nanoparticles are selected from the group consisting of Au, Ag, Cu, Pt, Ag/Au, Pt/Au, Cu/Au coreshell and alloy particles. In a more preferred embodiment, an Ag/AAO substrate (silver-filled porous anodic aluminum oxide nanochannels are provided by Prof. Yuh-Lin Wang) is employed.

Said mounting solution keeps the microorganism from moving and maintains it alive in an aqueous environment. In a preferred embodiment, the mounting solution is selected from the group consisting of agarose gel and glycerol. In one embodiment, the mounting solution is agarose gel in concentration of 0.05% ~ 5%, preferably 0.1% ~ 3%, more preferably 0.2% ~ 1.5% and most preferably 0.3% ~ 0.8%.

In the invention, said profile can be applied to identification of microorganism with species or strains with the aid of Pattern Recognition System.

In one embodiment, the microorganism is a bacterium, a fungus, or a cell.

In the invention, said profile can be applied to distinguish a Gram-positive bacterium from a Gram-negative bacterium by their specific fingerprint on Raman shift 400 cm⁻¹ to 1600 cm⁻¹. Preferably, the discriminative ranges are 500 cm⁻¹ to 900 cm⁻¹ and 1100 cm⁻¹ to 1500 cm⁻¹. Further, this goal can be reached by comparing the SERS spectrum of the unknown bacterium with an already known one. The present invention also relates to a method for detecting morphology alteration of a microorganism due to an antimicrobial agent or an infectious agent based on SERS, comprising:
(a) placing the microorganism on a SERS-active substrate;
(b) mounting the microorganism with a mounting solution;
(c) treating the microorganism with a pharmaceutically effective amount of the antimicrobial agent or the infectious agent;
(d) obtaining a SERS spectrum of the microorganism in step (c); and
(e) identifying the effect of the antimicrobial agent or the infectious agent by at least one new peak in the SERS spectrum in step (d) compared to a SERS spectrum of a control sample, wherein the control sample is not treated with the antimicrobial agent or the infectious agent.

In a preferred embodiment, the SERS-active substrate is a solid film embedded or covered with nanoparticles. Preferably, the nanoparticles are selected from the group consisting of Au, Ag, Cu, Pt, Ag/Au, Pt/Au, Cu/Au coreshell and alloy particles. In a more preferred embodiment, a Ag/AAO substrate (silver-filled porous anodic aluminum oxide nanochannels are provided by Prof.

Yuh-Lin Wang) is employed.

Said mounting solution keeps the microorganism from moving and maintains it alive in an aqueous environment. In a preferred embodiment, the mounting solution is selected from the group consisting of agarose gel and glycerol. In one embodiment, the mounting solution is agarose gel in concentration of 0.05% ~ 5%, preferably 0.1% ~ 3%, more preferably 0.2% ~ 1.5% and most preferably 0.3%~0.8%.

In one embodiment, the microorganism is a bacterium, a fungus, or a cell.

In one embodiment, the antimicrobial agent is selected from the group consisting of ampicillin, bacitracin, cephalosporin, chloramphenicol, erythromycin, hygromycin, kanamycin, methotrexate, neomycin, penicillin, polymynix, sulfonamide, tetracycline and zeocine.

In one embodiment, the infectious agent is a virus or an infectious bacterium.

Said method can be applied to development and screening of new antibiotics.

Said method can be applied to predicting the antibiotic mechanism of an antimicrobial agent or an infectious agent by examining where the change of relative intensity happens in the SERS spectrum compared to a SERS spectrum of a control sample.

The invention further relates to an apparatus for producing a profile of a microorganism based on surface enhanced Raman scattering (SERS), comprising:
(a) means of placing the microorganism on a SERS-active substrate;
(b) means of mounting the microorganism;
(c) means of obtaining a SERS spectrum of the microorganism in step (b);
   and
(d) means of analyzing the SERS spectrum to produce a profile of the microorganism.

The invention further relates to an apparatus for detecting morphology alteration of a microorganism due to an antimicrobial agent or an infectious agent based on surface enhanced Raman scattering (SERS), comprising:
(a) means of placing the microorganism on a SERS-active substrate;
(b) means of mounting the microorganism;
(c) means of treating the microorganism with a pharmaceutically effective amount of the antimicrobial agent or the infectious agent;
(d) means of obtaining a SERS spectrum of the microorganism in step (c);
   and
(e) means of identifying effect of the antimicrobial agent or the infectious agent by at least one new peak in the SERS spectrum in step (d) compared to a SERS spectrum of a control sample, wherein the control sample is not treated with the antimicrobial agent or the infectious agent.

The present invention clearly demonstrates that excellent signal-to-noise and reproducible Raman spectra of bacteria are obtained when the microorganism is placed on the novel substrate Ag/AAO. The SERS fingerprints clearly distinguish bacterial species. Besides, it can differentiate the spectra of bacteria treated with antibiotics. Therefore, SERS should be a powerful approach for the development and screening of new antibiotics.

The present invention provides a method for detecting *Mycobacterium tuberculosis* infection, comprising:
(a) placing the sample on a SERS-active substrate;
(b) mounting the microorganism with a mounting solution;
(c) obtaining a SERS spectrum of the sample in step (b);
(d) analyzing the SERS spectrum to produce a profile of the sample; and
(e) comparing the SERS spectrum of the sample with the SERS spectrum of gram-positive and gram-negative bacteria.

In one embodiment, the sample is blood, plasma, serum, phlegm, urine, cerebrospinal fluid, pleura fluid or tissue fluid. In further embodiment, the sample is blood, plasma, serum, phlegm or urine.

### EXAMPLE

The examples below are non-limiting and are merely representative of various aspects and features of the present invention.

### Example 1: Materials and Methods

### Bacterial sample and antibiotics preparation

Fourteen laboratory strains, Escherichia coli JM109, Escherichia coli JM109(mutant), Enterococcus faecalis 7-18uE, Enterococcus faecium 13631, Staphylococcus aureus 13649, Staphylococcus aureus 13615, Streptococcus agalactiae 13640, Enterococcus faecalis 13641 C2, Klebsiella pneumoniae 13641 C1, Proteus mirabilis 13621 C1, Enterobacter cloacae 13457 C2, E. coli 13594 C1, E. coli 13650, Escherichia coli JM109(mutant) and E. coli 13634 were analyzed. Bacteria were grown in 5mL of LB broth (~6 h) to OD600 = ~0.5, washed five times with PBS, and resuspended in 0.1 ml of PBS. About 3~5 µl of the bacteria suspension is placed on the Ag/AAO substrate. 0.5 % agarose gel was used to mount the bacterial samples for inhibiting the movement of bacteria. Five antibiotics: ampicillin, bacitracin, vancomycin, tetracycline and gentamycin were used in the examples. The former three could interfere with construction of the bacterial cell wall. The last two inhibited the protein synthesis in bacteria via disrupting the binding between tRNA and ribosome.

### Raman microscopy and highly raman-enhancing substrates

A Jobin Yvon Raman microscope (model LabRAM HR800) was used to observe the scattering excited by a 632.8 nm He-Ne laser. The spectral data acquisition time was in the 1-10s range and the SERS spectra were obtained with a 100x microscope objective for excitation/collection. The Ag/AAO substrate was the SERS-active substrate made of an array of Ag nanoparticles, which partially embedded in anodic aluminum oxide nanochannels. This substrate exhibited an ultrahigh Raman signal enhancement factor. (H. H. Wang, C. Y. Liu, S. B. Wu, N. W. Liu, C. Y. Peng, T. H. Chan, C. F. Hsu, J. K. Wang, and Y. L. Wang, Adv. Mater., 2006, 18, 491-495)

### Signal analysis

All signals got from SERS were then analyzed by Pattern Recognition System, one kind of machine learning, which aimed to classify data (patterns) based on either a priori knowledge or on statistical information extracted from the patterns. It consisted of a sensor that gathered the observations to be classified or described like SERS; a feature extraction mechanism that computed numeric or symbolic information from the observations; a classification or description scheme that did the actual job of classifying or describing observations; and an error estimation mechanism that estimated the efficiency of classifying and assisted proving the current clustering method.

The feature extraction method adopted in this invention comprising: (a) normalizing each sample by means of SERS from 2090 original data and to be summarized in to 1.0 vector; (b) taking logarithm of the data resulted from step (a); (c) normalizing the sum of the strongest signal parts within 2090 data as 1.0 vector; and (d) taking the most discriminative part resulted from the data of step (c).

The classification methods adopted in this invention were K-means algorithm and Support Vector Machine.

### Example 2: Producing Profiles Identifying Different Strains of Bacteria Using SERS

As showed in Figure 1, the SERS spectra of the strain *Escherichia coli* JM109 and *Escherichia coli* JM109₍ₘᵤₜₐₙₜ₎ were obtained. The spectra of the wild-type strain JM109 and the mutant strain JM109₍ₘᵤₜₐₙₜ₎ were significantly different from each other, demonstrating the strain specificity of SERS on detection and identification of bacterial cells of different strains.

### Example 3: Producing Profiles Identifying Different Species of Bacteria Using SERS

As showed in Figure 2, the SERS spectra of the strain *Escherichia coli* JM109₍ₘᵤₜₐₙₜ₎ and *Enterococcus faecalis* 7-18uE were obtained. The spectra of *Escherichia coli* JM109₍ₘᵤₜₐₙₜ₎ and *Enterococcus faecalis* 7-18uE were apparently different from each other, demonstrating the species specificity of SERS on detection and identification of bacterial cells of different species.

### Example 4: Producing Profiles Identifying Gram-positive and Gram-negative Bacteria

As showed in Figure 3, the SERS spectra of Gram-positive bacteria *(Enterococcus faecium* 13631, *Staphylococcus aureus* 13649, *Staphylococcus aureus* 13615, *Streptococcus agalactiae* 13640, *Enterococcus faecalis* 7-18uE, *Enterococcus faecalis* 13641 C2) and Gram-negative bacteria *(Escherichia coli* JM109_{(wild-type)}, *Klebsiella pneumoniae* 13641 C1, *Proteus mirabilis* 13621 C1, *Enterobacter cloacae* 13457 C2, *E. coli* 13594 C1, *E. coli* 13650, *Escherichia coli* JM109₍ₘᵤₜₐₙₜ₎. *E. coli* 13634) were obtained. The spectra of Gram-positive bacteria and Gram-negative bacteria were apparently different from each group, demonstrating the species specificity of SERS on discrimination of bacterial cells of different species.

### Example 5: Serial Detection of the SERS spectra of Gram-positive and Gram-negative Bacteria

To examine the stability of SERS profile whether change by time, the serial detection of the SERS spectra of Gram-positive bacteria *(Streptococcus agalactiae* 13640 and *Staphylococcus aureus* 13615) and Gram-negative bacteria (*Enterobacter cloacae* 13457 C2 and *Proteus mirabilis* 13621 C1) were obtained at various time points and shown in Figure 4. The results demonstrated that the SERS profile was stable least for 59 minutes and also the signals of relative intensity.

### Example 6: Monitoring the Effects of Antibiotics Ampicillin and Bacitracin on Bacteria Using SERS

To identify the effects of antibiotics on bacteria, the antibiotic susceptible strain *Escherichia coli* JM109 was treated with 10 µg/ml ampicillin for 35 min after being mounted with 0.5 % agarose gel. The spectrum of JM109 treated with ampicillin was apparently different with that of JM109 with no treatment (Figure 5). The presence of a new peak at 700 cm⁻¹ in the spectrum of JM109 treated with ampicillin demonstrated the oscillation of a new molecular bond caused by ampicillin.

To further identify the effects of antibiotics on bacteria, another antibiotic susceptible strain *Escherichia coli* JM109₍ₘᵤₜₐₙₜ₎ was treated with 25 µg/ml bacitracin for 48 min after being mounted with 0.5 % agarose gel. The spectrum of JM109₍ₘᵤₜₐₙₜ₎ treated with bacitracin was apparently different with that of JM109₍ₘᵤₜₐₙₜ₎ with no treatment (Figure 6). The presence of a new peak at 700 cm⁻¹ in the spectrum of JM109₍ₘᵤₜₐₙₜ₎ treated with bacitracin demonstrated the oscillation of a new molecular bond caused by bacitracin.

The new peak at 700 cm⁻¹ occurred in both of the results shown in Figures 3 and 4 demonstrated that both of cell wall-active antibiotics, ampicillin and bacitracin, did interfere with the construction of the bacterial wall and induce the changes of cell wall conformation.

### Example 7: Monitoring the Effects of Antibiotic Vancomycin on Bacteria Using SERS

To identify the effect of other cell wall-active antibiotic on other strain of bacteria, the antibiotic susceptible strain *Staphylococcus aureus* 13649 was treated with 25 µg/ml vancomycin for 54 min after being mounted with 0.5 % agarose gel. The presence of new peaks at 800 cm⁻¹ to 1500 cm⁻¹ demonstrated that vancomycin, a cell wall-active antibiotic, interfered with the construction of the bacterial wall and induced the changes of cell wall conformation (Figure 7).

### Example 8: Monitoring the Effects of Antibiotic Tetracycline on Bacteria Using SERS

To identify the effect of antibiotic on bacteria, the antibiotic susceptible strain *Escherichia coli* JM109₍ₘᵤₜₐₙₜ₎ was treated with 50 µg/ml tetracycline after being mounted with 0.5% agarose gel. The resulted spectra were obtained at various time points (Figures 8 and 9). Different new peaks were observed at different time points, demonstrating the oscillation of different new molecular bonds at different time points. The phenomenon perfectly reflected the dynamic metabolism after the inhibition of protein synthesis by tetracycline.

### Example 9: Monitoring the Effects of Antibiotic Gentamycin on Bacteria Using SERS

To identify the effect of other protein synthesis-interfered antibiotic on other strain of bacteria, the antibiotic susceptible strain E. *coli* 13650 was treated with 25 µg/ml gentamycin for 37 min after being mounted with 0.5 % agarose gel. The presence of new peaks at 418 cm⁻¹ and at 800 cm⁻¹ to 1500 cm⁻¹ demonstrated that gentamycin induced morphological change of the bacterium may caused by inhibition of protein synthesis (Figure 10).

### Example 10: Monitoring the Effects of Antibiotic on Drug-Resistant Strains Using SERS

To confirm the effects of antibiotics on bacteria, the antibiotic resistant strain *Enterococcus faecalis* 7-18uE was treated with 10 µg/ml ampicillin for 35 min or with 25 µg/ml bacitracin for 48 min after being mounted with 0.5 % agarose gel. In contrast with the spectra of antibiotic susceptible strains *Escherichia coli* JM109 and *Escherichia coli* JM109₍ₘᵤₜₐₙₜ₎, no changes in the spectra were observed in the antibiotic resistant strain *Enterococcus faecalis* 7-18uE treated with antibiotics (Figure 11).

### Example 11: Detecting the Mycobacterium tuberculosis by Using SERS Spectra

Following the steps of Example 1, the test could be applied to examine TB.

As showed in Figure 12, the SERS spectra of the gram-positive bacteria, the gram-negative bacteria and Mycobacterium were obtained. The Mycobacterium apparently was different from others.

### Example 12: Detecting Different Growth States of Gram-negative Bacteria by Using SERS Spectra

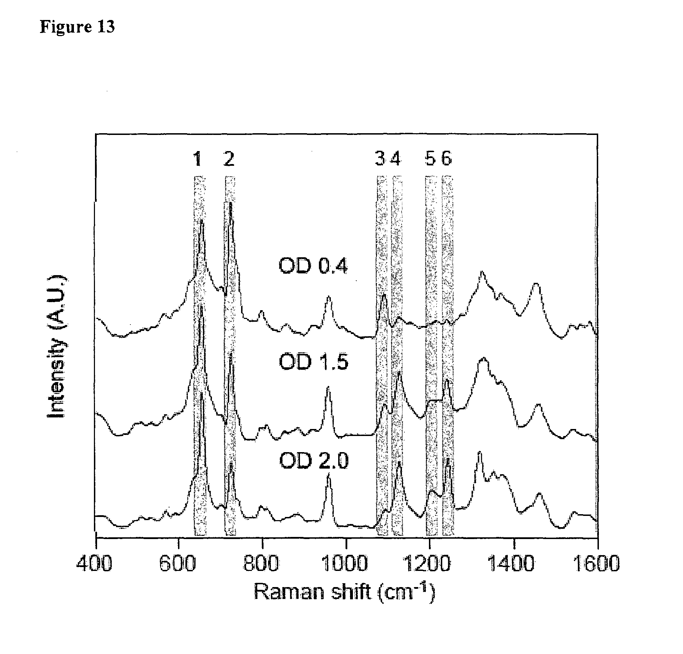

To indentify different growth states of gram-negative bacteria, *Escherichia coli* at the beginning and middle of the exponential phase and the stationary phase were measured (OD₆₀₀= 0.4, 1.5, 2.0 respectively). The SERS spectra obtained from the bacteria growing at the start of exponential phase (OD₆₀₀= 0.4), approaching (OD₆₀₀= 1.5) or at the stationary phase (OD₆₀₀= 2.0), exhibited characteristic changes in SERS profiles (Figure 13). The intensities of peaks at 655 cm⁻¹ (#1), 1130 cm⁻¹ (#4), 1219 cm⁻¹ (#5), and 1245 cm⁻¹ (#6) progressively increased as the bacteria grown from exponential to stationary phase, while the intensities of the peaks at 725 cm⁻¹ (#2) and 1095 cm⁻¹ (#3) gradually decreased.

One skilled in the art readily appreciates that the present invention is well adapted to carry out the objects and obtain the ends and advantages mentioned, as well as those inherent therein. The bacterial strains, cell lines, antibiotics, mounting solutions, agents, SERS-active substrates, equipments, processes and methods for producing them are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the scope and spirit of the invention. All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations, which are not specifically disclosed herein. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Other embodiments are set forth within the following claims.

## Claims

1. A method for producing a profile identifying a microorganism based on surface enhanced Raman scattering (SERS), comprising:
(a) placing the microorganism on a SERS-active substrate;
(b) mounting the microorganism with a mounting solution;
(c) obtaining a SERS spectrum of the microorganism in step (b); and
(d) analyzing the SERS spectrum to produce a profile of the microorganism.

2. The method of claim 1, wherein the SERS-active substrate is a solid film embedded or covered with nanoparticles.

3. The method of claim 2, wherein the nanoparticles are selected from the group consisting of Au, Ag, Cu, Pt, Ag/Au, Pt/Au, Cu/Au coreshell and alloy particles.

4. The method of claim 1, wherein the mounting solution keeps from moving and maintains the microorganism alive in an aqueous environment.

5. The method of claim 1, wherein the mounting solution is selected from the group consisting of agarose gel and glycerol.

6. The method of claim 1, wherein the profile is applied to identification of microorganism with species or strains.

7. The method of claim 1, wherein the profile is produced by means of Pattern Recognition System.

8. The method of claim 1, wherein the microorganism is a bacterium, a fungus, or a cell.

9. The method of claim 1, wherein the SERS spectrum differentiates a Gram-positive bacterium from a Gram-negative bacterium.

10. The method of claim 9, wherein the discriminative points are range from Raman Shift 500 cm⁻¹ to 900 cm⁻¹ and from 1100 cm⁻¹ to 1500 cm-1.

11. The method of claim 9, which further comprises a method comparing the SERS spectrum of the unknown bacterium with an already known one.

12. A method for detecting morphology alteration of a microorganism due to an antimicrobial agent or an infectious agent based on surface enhanced Raman scattering (SERS), comprising:
(a) placing the microorganism on a SERS-active substrate;
(b) mounting the microorganism with a mounting solution;
(c) treating the microorganism with a pharmaceutically effective amount of the antimicrobial agent or the infectious agent;
(d) obtaining a SERS spectrum of the microorganism in step (c); and
(e) identifying the effect of the antimicrobial agent or the infectious agent by at least one new peak in the SERS spectrum in step (d) compared to a SERS spectrum of a control sample, wherein the control sample is not treated with the antimicrobial agent or the infectious agent.

13. The method of claim 12, wherein the SERS-active substrate is a solid film embedded or covered with nanoparticles.

14. The method of claim 12, wherein the mounting solution keeps the microorganism from moving and maintains the microorganism alive in an aqueous environment.

15. The method of claim 12, wherein the mounting solution is selected from the group consisting of agarose gel and glycerol.

16. The method of claim 12, wherein the microorganism is a bacterium, a fungus, or a cell.

17. The method of claim 12, wherein the antimicrobial agent is selected from the group consisting of ampicillin, bacitracin, cephalosporin, chloramphenicol, erythromycin, hygromycin, kanamycin, methotrexate, neomycin, penicillin, polymynix, sulfonamide, tetracycline and zeocine.

18. The method of claim 12, wherein the infectious agent is a virus or an infectious bacterium.

19. The method of claim 12, which is applied to development and screening of new antibiotics.

20. The method of claim 12, which is applied to predicting the antibiotic mechanism of an antimicrobial agent or an infectious agent by examining where the change of relative intensity happens in the SERS spectrum compared to a SERS spectrum of a control sample.

21. An apparatus for producing a profile of a microorganism based on surface enhanced Raman scattering (SERS), comprising:
(a) means of placing the microorganism on a SERS-active substrate;
(b) means of mounting the microorganism;
(c) means of obtaining a SERS spectrum of the microorganism in step (b);
and
(d) means of analyzing the SERS spectrum to produce a profile of the microorganism.

22. An apparatus for detecting morphology alteration of a microorganism due to an antimicrobial agent or an infectious agent based on surface enhanced Raman scattering (SERS), comprising:
(a) means of placing the microorganism on a SERS-active substrate;
(b) means of mounting the microorganism;
(c) means of treating the microorganism with a pharmaceutically effective amount of the antimicrobial agent or the infectious agent;
(d) means of obtaining a SERS spectrum of the microorganism in step (c);
and
(e) means of identifying effect of the antimicrobial agent or the infectious agent by investigating the signal alteration in the SERS spectrum in step (d) compared to a SERS spectrum of a control sample, wherein the control sample is not treated with the antimicrobial agent or the infectious agent.

23. The apparatus of claim 24, wherein the step (e) is carried out with the aids of Pattern Recognition System.

24. A method for detecting Mycobacterium tuberculosis infection, comprising:
(a) placing the sample on a SERS-active substrate;
(b) mounting the microorganism with a mounting solution;
(c) obtaining a SERS spectrum of the sample in step (b);
(d) analyzing the SERS spectrum to produce a profile of the sample; and
(e) comparing the SERS spectrum of the sample with the SERS spectrum of gram-positive and gram-negative bacteria.

25. The method of claim 26, wherein the sample is blood, plasma, serum, phlegm, urine, cerebrospinal fluid, pleura fluid or tissue fluid.
